# EUROPÄISCHE PATENTSCHRIFT

(11) **EP 1 788 963 B1**
(45) Veröffentlichungstag und Bekanntmachung des Hinweises auf die Patenterteilung: **02.11.2011**
(21) Anmeldenummer: 05774070.6
(22) Anmeldetag: 19.08.2005
(51) Int. Cl.: A61B 18/12, A61B 18/14

(54) **VORRICHTUNG ZUM ELEKTROCHIRURGISCHEN VERÖDEN VON KÖRPERGEWEBE**
DEVICE FOR THE ELECTRO-SURGICAL SCLEROSING OF BODY TISSUES
DISPOSITIF DE SCLEROSE ELECTROCHIRURGICALE DE TISSUS CORPORELS

(30) Priorität: 20.08.2004 DE 102004041681
(43) Veröffentlichungstag der Anmeldung: 30.05.2007
(73) Patentinhaber: Celon AG Medical Instruments, 14513 Teltow (DE)
(72) Erfinder: DESINGER, Kai, 12157 Berlin (DE); ROGGAN, André, 12163 Berlin (DE); STEIN, Thomas, 14513 Teltow (DE); PREZEWOWSKY, Thomas, 14513 Teltow (DE)
(74) Vertreter: Eisenführ, Speiser & Partner
(86) Internationale Anmeldenummer: PCT/EP2005/054107
(87) Internationale Veröffentlichungsnummer: WO 2006/021550

(56) Entgegenhaltungen:
- WO-A-94/24949
- DE-A1- 3 120 102
- DE-A1- 10 224 154
- DE-A1- 19 541 566
- US-A- 5 540 684
- US-A1- 2002 151 884
- GOLDBERG S N: "Radiofrequency tumor ablation: principles and techniques." EUROPEAN JOURNAL OF ULTRASOUND : OFFICIAL JOURNAL OF THE EUROPEAN FEDERATION OF SOCIETIES FOR ULTRASOUND IN MEDICINE AND BIOLOGY. JUN 2001, Bd. 13, Nr. 2, Juni 2001 (2001-06), Seiten 129-147, XP002356025 ISSN: 0929-8266

## Beschreibung

Die vorliegende Erfindung betrifft eine Vorrichtung für die Applikation eines Hochfrequenzstromes zum thermischen Veröden von Körpergewebe. Die Applikationsvorrichtung umfasst mindestens zwei Elektroden, die im Anwendungsfall in Kontakt mit dem Körpergewebe stehen und von denen mindestens eine Elektrode in das Körpergewebe eingeführt ist. Außerdem umfasst die Applikationsvorrichtung einen Hochfrequenzgenerator zum Erzeugen einer Hochfrequenzspannung, der mit mindestens zwei der Elektroden verbunden ist, eine Messvorrichtung zum Messen der Impedanz bzw. des ohmschen Widerstands zwischen den ausgewählten Elektroden, sowie eine Kontrolleinrichtung, die bei Bedarf die elektrische Ausgangsleistung des Hochfrequenzgenerators verändert und in einer bevorzugten Variante zusätzlich eine externe Pumpe steuern kann.

Die Ausgangsleistung des Hochfrequenzgenerators ergibt sich, wenn die an den Ausgängen des Hochfrequenzgenerators anliegende Hochfrequenzspannung im Rahmen einer Hochfrequenzstromapplikation an die mit dem Körpergewebe in Kontakt stehenden Elektroden abgeben wird und einen Hochfrequenzstrom durch das Körpergewebe hervorruft.

Das elektrochirurgische, insbesondere das elektrothermische Veröden von pathologisch verändertem Gewebe, im folgenden kurz Gewebe oder Körpergewebe genannt, ist eine in der Medizin bekannte Methode. Von besonderem Interesse ist diese Methode für die Therapie von Organtumoren, zum Beispiel Lebertumoren. Zur Verödung werden eine oder mehrere Elektroden im zu verödenden Gewebe, d. h. dem Tumorgewebe, oder in dessen unmittelbarer Nähe platziert und ein Wechselstrom zwischen den Elektroden oder einer Elektrode und einer außen am Körper fixierten Neutralelektrode fließen lassen. Fließt der Strom zwischen der Elektrode und der Neutralelektrode (ggf. auch zwischen mehreren Elektroden und einer oder mehreren Neutralelektroden), so spricht man von einer monopolaren Elektrodenanordnung. Fließt der Strom dagegen zwischen den im Gewebe befindlichen Elektroden selbst (in diesem Fall müssen im Gewebe mindestens zwei Elektroden vorhanden sein), so spricht man von einer bipolaren Anordnung. Von einer multipolaren Anordnung spricht man, wenn im Gewebe mehr als zwei Elektroden vorhanden sind, zwischen denen der Wechselstrom abwechselnd fließt.

Die zum platzieren im Gewebe vorgesehenen Applikatoren sind in der Regel als Elektrodennadeln oder als flexible Elektrodenkatheter ausgebildet. Elektrodennadeln besitzen einen elektrisch leitenden zylinderförmigen Schaft, der mit Ausnahme eines oder mehrerer distaler Bereiche, den sogenannten aktiven Bereichen, die jeweils eine (aktive) Elektrode bilden, gegen das umliegende Gewebe elektrisch isoliert ist. Flexible Elektrodenkatheter besitzen einen biegeweichen, nicht leitenden Schaft und einen oder mehrere distale aktive Bereiche, die jeweils eine (aktive) Elektrode bilden. Die aktiven Elektroden stehen mit dem Körpergewebe in elektrisch leitfähiger Verbindung. Optional sind die aktiven Elektroden auch mit integrierten Thermosensoren ausgestattet. In speziellen Ausführungsformen können am distalen Ende des Schaftes weitere aktive Elektroden mechanisch ausgefahren werden, um das therapierbare Gewebevolumen zu vergrößern.

Um die Effizienz der Behandlung zu verbessern sind auch Applikatoren mit Elektrodennadeln bekannt, welche einen internen, geschlossenen Kühlkreislauf besitzen, welcher durch eine externe Pumpe betrieben wird (interne Kühlung). Ebenfalls bekannt sind Elektrodennadeln, die in ihrem distalen Bereich eine oder mehrere Öffnungen besitzen, um mittels einer externen Pumpe kleine Flüssigkeitsmengen in das zu behandelnde Körpergewebe einzubringen (offene Spülung).

Mittels Hochfrequenzgenerator wird in der monopolaren Anordnung ein Stromfluss zwischen den aktiven Elektroden und der bzw. den Neutralelektroden induziert. In der alternativen bipolaren Anordnung kann auf die Neutralelektroden verzichtet werden. Der Stromkreis wird in diesem Fall über eine weitere aktive Elektrode geschlossen, wobei die erforderlichen aktiven Elektroden in einer koaxialen Anordnung gegeneinander isoliert auf der Elektrodennadel oder auf zwei getrennten Elektrodennadeln angeordnet sein können.

Durch den ohmschen Gewebewiderstand, der ein Teil der komplexen Gewebeimpedanz ist, erfolgt eine Umsetzung des über die Elektroden applizierten Wechselstroms in joulsche Wärme. Bei Temperaturen zwischen 50°C und 100°C kommt es zu einer massiven Denaturierung der körpereigenen Proteine (Koagulation) und in der Folge zum Absterben der betroffenen Gewebeareale. Aufgrund der hohen Stromdichte an den aktiven Elektroden erfolgt die Erwärmung vorwiegend im Bereich dieser Elektroden, so dass eine lokale thermische Tumordestruktion möglich ist.

Eine Vorrichtung und ein Verfahren zum elektrothermischen Veröden pathologischen Gewebes ist beispielsweise in US 5,630,426 offenbart.

Entscheidend für eine effektive und vor allem sichere Therapie ist die Ausbildung einer thermischen Destruktionszone, die der Ausdehnung des pathologischen Gewebes, d. h. des Tumorgewebes, optimal angepasst ist.

Eine Weiterentwicklung der monopolaren bzw. bipolaren Gewebeverödung ist in der PCT/EP03/05439 beschrieben, bei der mehr als zwei Elektroden in das Körpergewebe eingebracht werden und anhand einer Auswahleinrichtung für ein bestimmtes Zeitintervall jeweils mindestens zwei Elektroden mit Hochfrequenzspannung versorgt werden. Die Auswahl geeigneter Elektrodenpaarungen erfolgt hierbei anhand der zwischen allen möglichen Elektrodenpaarungen gemessenen Impedanz oder dem gemessenen ohmschen Widerstand. Diese Ausführungsform ist besonders vorteilhaft, weil hiermit besonders effektiv auch große Gewebevolumina thermisch verödet werden können, was z. B. eine zuverlässige Tumortherapie ermöglicht.

Es ist eine bekannte Tatsache, dass die Impedanz bzw. der ohmsche Gewebewiderstand stark vom Fortschreiten der thermischen Gewebezerstörung abhängt, weshalb diese Parameter zur Therapiesteuerung genutzt werden können. So weisen die Impedanz und der ohmsche Gewebewiderstand im Verlauf der Gewebeverödung ein typisches zeitliches Verhalten auf.

Werden zwei Elektroden in Körperkontakt gebracht, stellt sich zunächst ein Impedanz- bzw. ohmscher Widerstandswert ein, der sowohl von der Elektrodengeometrie (Oberfläche) und dem Elektrodenabstand abhängt, als auch durch das zwischen den Elektroden liegende Gewebe und dessen Leitfähigkeit beeinflusst wird. Darüber hinaus ist zu berücksichtigen, dass bei Betrachtung der Gewebeimpedanz noch zusätzliche kapazitive und induktive Einflüsse durch die Verbindungskabel zwischen Hochfrequenzgenerator und Elektroden zu berücksichtigen sind. Dadurch wird eine präzise Regelung des Koagulationsprozesses erschwert. Dies macht insbesondere den ohmschen Widerstand zu einem Parameter, der von solch parasitären Einflüssen nahezu frei ist und daher vorteilhafter als die Impedanz für die Regelung der Koagulation verwendet werden kann. Ohne Einschränkung des Erfindungsgedankens wird im weiteren Verlauf nur noch vom Widerstand gesprochen, da die vorgeschlagene Vorrichtung sowohl die Impedanz als auch den ohmsche Widerstand zur Therapiesteuerung heranziehen kann.

Nach dem Aktivieren der Hochfrequenzspannung an mindestens zwei Elektroden, zwischen denen sich Körpergewebe im elektrischen Kontakt befindet, sinkt der Gewebewiderstand zunächst signifikant unter den Ausgangswert. Dies ist bedingt durch die beginnende Gewebeerwärmung und die damit verbundene bessere Beweglichkeit der den elektrischen Stromfluss vermittelnden Ionen im Körpergewebe (z. B. Na⁺, Cl⁻). Darüber hinaus kommt es bei weiterer Temperaturerhöhung zu Rupturen der Zellmembran und damit ebenfalls zu einer gesteigerten Leitfähigkeit der den Stromfluss vermittelnden Ionen.

Wird jedoch im Körpergewebe eine Temperatur von ca. 100°C erreicht, beginnt das Gewebewasser in die Dampfphase überzutreten. Die genaue Temperatur des Siedepunktes hängt dabei von dem Druck ab, der im Gewebe aufgebaut werden kann, bevor der Dampf entweichen kann, und liegt typischerweise nicht wesentlich über 100°C. Aufgrund der mit dem Übertritt des Wassers von der Flüssigphase in die Dampfphase verbundenen Ausdehnung entfernt sich der Dampf durch Gewebespalten von den elektrodennahen Bereichen, in denen typischerweise die höchsten Temperaturen zu verzeichnen sind. Diese sogenannte Vaporisation geht somit einher mit einer Verringerung des Wassergehaltes in jenen elektrodennahen Bereichen, so dass es allmählich zu einer Austrocknung des Gewebes kommt. Zunächst reicht das restliche in den elektrodennahen Bereichen verbliebene Zellwasser noch aus, um die Beweglichkeit der den Stromfluss vermittelnden Ionen zu gewährleisten, jedoch wird mit zunehmendem Austrocknungsgrad die Beweglichkeit der Ionen mehr und mehr eingeschränkt. Dies ist der Zeitpunkt, an dem der Gewebewiderstand signifikant ansteigt.

Damit kommt dann eine Art Kettenreaktion in Gang, bei der durch den elektrodennahen, austrocknungsbedingten Widerstandsanstieg im Verhältnis zur gesamten elektrischen Stromverteilung bei gleich bleibender Ausgangsleistung mehr Energie in diesen elektrodennahen Bereichen umgesetzt wird, so dass sich die Vaporisation und damit auch die Austrocknung beschleunigt. Als Folge ist ein exponentieller Anstieg des Widerstandes zu verzeichnen.

Das so beschriebene Verhalten wirkt sich bekanntermaßen negativ auf die eigentliche Therapiemaßnahme Gewebeverödung aus, weil ein frühes Einsetzen der Austrocknung insbesondere bei sehr hohen Leistungsdichten das Ausbreiten einer adäquaten, d. h. großvolumigen thermischen Destruktionszone verhindert. Dies ist dadurch begründet, dass Hochfrequenzgeneratoren nach dem Stand der Technik einer Leistungskennlinie folgen, die nur in einem bestimmten Impedanz bzw. Widerstandsbereich (dem Anpassungsfall) eine optimale Leistungsabgabe ermöglichen. Liegen die Impedanzen bzw. ohmsche Widerstände außerhalb dieses Bereiches, was zum Beispiel bei einer elektrodennahen Gewebeaustrocknung der Fall ist, kann keine ausreichende Leistung mehr in das Gewebe abgegeben werden und der Verödungsvorgang kommt zum Stillstand. Dies ist jedoch unter allen Umständen zu verhindern, weil sonst eine unvollständige Tumortherapie mit dem Risiko dem Wiederauftreten der Erkrankung am gleichen Ort die Folge sein könnte.

In begrenztem Maße können intern gekühlte Applikatoren durch ihre niedrigere Gewebekontakttemperatur den Vorgang der Austrocknung hinauszögern, jedoch nicht in dem Maße, wie es für eine effiziente Therapie erforderlich wäre. Auch offen gespülte Elektrodennadeln verzögern das Einsetzen der Austrocknung, indem ständig durch die externe Pumpe Flüssigkeit in das Austrocknungsgebiet nachgeliefert wird. Allerdings hat sich dieses Verfahren in der Praxis nicht bewährt, da das unkontrollierte Einbringen von Flüssigkeit insbesondere in Tumorgewebe mit einem erheblichen Zellverschleppungsrisiko einhergehen kann.

Um die vorzeitige Gewebeaustrocknung zu verhindern, ist daher bei den Hochfrequenzgeneratoren nach dem Stand der Technik eine Ausgangsleistung zu wählen, die die vorzeitige Austrocknung ausschließt, aber andererseits auch ausreichend ist, um die Gewebeverödung in einer angemessenen Zeit durchzuführen. Dies gestaltet sich in der klinischen Routine allerdings als äußerst problematisch, weil letztlich jedes Gewebe ganz spezifische Eigenschaften aufweist, die per se dem die Therapie durchführenden Personal nicht bekannt sind. Diese Gewebeeigenschaften umfassen neben den physikalischen Parametern spezifischer Gewebewiderstand, Wärmeleitfähigkeit und Wärmekapazität vor allem den lokalen Blutfluss, der aufgrund seiner kühlenden Wirkung den Verödungsprozess maßgeblich mitbestimmt.

Somit kommt es in der klinischen Praxis sehr häufig zu einer fehlangepassten Leistungseinstellung. Diese führt entweder bei Unterleistung zu einer unnötigen Verlängerung der Therapie oder aber bei der wesentlich kritischeren Überleistung zu einem vorzeitigen Therapieabbruch aufgrund eines austrocknungsbedingten Widerstandsanstiegs. Die einzige Möglichkeit, die Therapie doch noch erfolgreich zu beenden besteht darin, nach dem Feststellen der Gewebeaustrocknung die Leistungsabgabe für einen bestimmten Zeitraum zu unterbrechen, bis sich wieder ausreichend Gewebeflüssigkeit in den elektrodennahen Bereichen angesammelt hat.

Wird nämlich die Leistungsabgabe unterbrochen, sinkt die Temperatur aufgrund des großen Temperaturgradienten schnell unter die Verdampfungstemperatur, so dass Dampf an den Elektroden kondensieren kann. Wesentlich wichtigerer ist jedoch die Tatsache, dass aufgrund des geringen Wassergehaltes an den Elektroden und dem hohen Wassergehalt in der Umgebung ein großer Gradient des Wassergehaltes vorliegt, so dass relativ schnell Wasser zurückdiffundiert und so den lokalen Wassergehalt an den Elektroden wieder erhöht. In gleichem Maße verringert sich der Widerstand der nach Leistungsunterbrechung einen exponentiell fallenden Verlauf zeigt, um schließlich in einen konstanten Wert geringfügig unterhalb des ursprünglichen Ausgangswertes überzugehen, was die ausreichende Rückdiffusion von Gewebewasser signalisiert. Es ist jedoch per se nicht bekannt, wie lange der Prozess der Rückdiffusion dauert bis der konstante Widerstandswert erreicht wird und ein Fortsetzen der Therapie mit erneuter Leistungsabgabe wieder möglich ist.

Bei schlechter, sprich zu hoher Leistungsvorwahl, muss das Prozedere der Leistungsunterbrechung mitunter mehrfach während einer Therapie wiederholt werden, um die Therapie sicher abschließen zu können.

Es sind bereits Vorrichtungen bekannt, die das klinische Vorgehen in gewisser Weise vereinfachen. So ist beispielsweise in Goldberg et al. [Goldberg SN et al.: Percutaneous radiofrequency tissue ablation: optimization of pulsedradiofrequency technique to increase coagulation necrosis; J. Vasc. Interv. Radiol. 10(7):907-16, 1999.] eine Vorrichtung beschrieben, bei der die Impedanz beim Start der Verödung gemessen und gespeichert wird. Sobald die Impedanz im Verlauf der Verödung den Ausgangswert um einen festen Wert überschreitet, wird die Leistung für ein festes Zeitintervall unterbrochen, um anschließend wieder automatisch den ursprünglichen Ausgangswert anzunehmen.

Dieses Verfahren weist jedoch entscheidende Nachteile auf. Zum einen wird eine feste Impedanzschwelle für das Auslösen der Leistungsabschaltung angenommen. Damit wird aber die Auslöseempfindlichkeit vom Absolutwert der Ausgangsimpedanz abhängig. Bei kleinen Ausgangsimpedanzen (z. B. in Lebergewebe) kann die Auslösung zu spät erfolgen, bei hohen Ausgangsimpedanzen (z. B. in Lungengewebe) kann die Auslösung im Gegensatz dazu zu früh erfolgen, weil bereits kleine relative Änderungen der Impedanz zum Überschreiten der Auslöseschwelle führen. Damit wird aber die Therapieeffizienz verschlechtert, weil die Phase ohne Leistungsabgabe zu früh einsetzt. Ein weiterer Nachteil ist die Tatsache, dass die Leistung nach dem Erkennen eines Überschreitens der Impedanzschwelle für ein festes Zeitintervall unterbrochen werden muss. Ob das Intervall ausreicht, um genügend Wasser zurückdiffundieren zu lassen ist unbekannt. War das Intervall zu lang, geht Therapieeffizienz verloren, war es zu kurz, setzt sofort wieder Austrocknung ein und es geht ebenfalls Therapieeffizienz verloren.

DE 102 24 154 zeigt eine Vorrichrung gemäß der Preambel von Anspruch 1.

Aufgabe der vorliegenden Erfindung ist es, eine Vorrichtung zur Verfügung zu stellen, die bei jedem möglichen Impedanz- und Widerstandsverhalten eine optimale Therapieeffizienz ermöglicht und sicher die Austrocknung des elektrodennahen Gewebes verhindert.

Diese Aufgabe wird durch eine Hochfrequenzstrom-Applikationsvorrichtung zur thermischen Verödung von Körpergewebe gemäß Anspruch 1 gelöst, die mindestens zwei Elektroden in Kontakt mit dem Körpergewebe umfasst. Im Fall einer bipolaren oder multipolaren Anordnung sind ein oder mehrere bipolare Applikatoren mit jeweils zwei Elektroden auf einem Schaft oder zwei oder mehrere Applikatoren mit nur einer Elektrode pro Schaft in das Gewebe einzuführen. Außerdem umfasst die Applikationsvorrichtung einen Hochfrequenzgenerator zum Erzeugen einer Hochfrequenzspannung, der mit mindestens zwei der Elektroden verbunden ist sowie eine Mess- und Rechenvorrichtung zum Messen der Impedanz bzw. des ohmschen Widerstands zwischen den ausgewählten Elektroden. Zur Vereinfachung werden im folgenden ohne Beschränkung des Erfindungsgedankens beide Begriffe Impedanz und ohmscher Widerstand kurz als Widerstand bezeichnet. Weiterhin enthält die Hochfrequenzstrom-Applikationsvorrichtung eine Kontrolleinrichtung zur Veränderung der elektrischen Ausgangsleistung des Hochfrequenzgenerators während einer Hochfrequenzstromapplikation.

In einer bevorzugten Ausführungsform ist die Mess- und Rechenvorrichtung ausgebildet, aus mindestens zwei der Parameter Stromstärke, Spannung, Phasenbeziehung und Wirkleistung des therapeutischen Hochfrequenzstromes den Widerstand zwischen den zwei Elektroden zu ermitteln, zwischen denen jeweils ein therapeutischer Hochfrequenzstrom fließt.

Vorzugsweise sind die zwei Elektroden, zwischen denen die Impedanz- oder ohmsche Widerstandsmessung erfolgt, auf einem Schaft angeordnet, der in das Körpergewebe einzuführen ist.

Eine der zwei Elektroden, zwischen denen die Impedanz- oder ohmsche Widerstandsmessung erfolgt, kann eine auf die Hautoberfläche aufzubringende Neutralelektrode sein. Vorzugsweise ist diese Neutralelektrode aus mehreren getrennten Elementen gebildet, die auf unterschiedliche Hautoberflächen aufzubringen sind und durch eine elektrisch leitende Verbindung auf demselben elektrischen Potenzial liegen.

Es ist jedoch auch möglich, für die Bestimmung des Gewebeverhaltens einen von der therapeutischen Spannungsquelle getrennten Stromkreis zu verwenden, der auch bei einer anderen als der Therapiefrequenz arbeiten kann und ebenfalls eine Kombination der Parameter Stromstärke, Spannung, Wirkleistung und Phasenverhalten zur Ermittlung des Widerstandes auswertet.

Wesentliches Merkmal der Vorrichtung ist es nun, dass zur Detektion der Gewebeaustrocknung nicht der Absolutwert des Widerstandes gemessen wird, sondern sein zeitliches Änderungsverhalten durch die Auswertung der ersten Ableitung des Widerstandes nach der Zeit ermittelt wird. Damit wird die Erkennung der Gewebeaustrocknung unabhängig vom Ausgangswert des Widerstandes und kann ohne Beschränkung für unterschiedliche Gewebearten, Elektrodengeometrien und -konfigurationen angewendet werden.

In einer bevorzugten Ausführungsform liefert die Messvorrichtung in kurzen Zeitabständen von einigen Millisekunden jeweils ein neues Ergebnis der Widerstandsmessung. Von diesem Ergebnis wird z. B. durch einen Mikroprozessor das Ergebnis der vorhergehenden Messung subtrahiert und durch die Länge des Zeitintervalls zwischen beiden Messvorgängen geteilt. Auf diese Weise wird die erste Ableitung der Impedanz oder des ohmschen Widerstands nach Art eines Differenzenquotieten ais jeweils zwei Messwerten für die Impedanz bzw. den ohmschen Widerstand gebildet. Ist das Ergebnis positiv und liegt der Wert oberhalb eines vorgegebenen Schwellwertes (im folgenden, insbesondere in der Figurenbeschreibung auch als Grenzwert bezeichnet), der typischerweise im Bereich 5 Ohm/s bis 50 Ohm/s liegen sollte, liefert die Messvorrichtung ein Signal an die Kontrolleinrichtung, dass anzeigt, dass die elektrodennahe Austrocknung unmittelbar bevorsteht. Die Kontrolleinrichtung reduziert daraufhin die Ausgangsleistung signifikant, um der Austrocknung entgegen zu wirken.

Die Kontrolleinrichtung ist vorzugsweise so ausgebildet, dass sie die Ausgangsleistung über einen kurzen Zeitraum kontinuierlich reduziert. Ein geeigneter Zeitraum ist zwischen 0,1 und 5 Sekunden lang.

Werden die zur Ermittlung des Widerstandes ausgewerteten Parameter aus dem therapeutischen Hochfrequenzstrom abgeleitet, sollte die reduzierte Ausgangsleistung klein genug sein, die weitere Gewebeaustrocknung zu verhindern und genügend Gewebewasser in die Austrocknungsbereiche zurückfließen zu lassen (die Temperatur an den Elektroden sollte deutlich unter 100°C sinken). Andererseits sollte die reduzierte Ausgangsleistung noch groß genug sein, um auch weiterhin eine sichere Bestimmung des Widerstandswertes aus dem Hochfrequenzstrom zu ermöglichen. Ein typischer Wert für die reduzierte Ausgangsleistung liegt im Bereich 2 % bis 50 % der ursprünglichen Ausgangsleistung. Wird dagegen der Widerstand unabhängig vom therapeutischen Hochfrequenzstrom ermittelt, kann die Ausgangsleistung sogar auf 0 % reduziert werden.

Ist die Messung des Widerstandes mit einem Rauschen behaftet, was insbesondere dann der Fall sein kann, wenn die zur Ermittlung des Widerstandes ausgewerteten Parameter aus dem therapeutischen Hochfrequenzstrom abgeleitet werden, ist es sinnvoll, die Messwerte einer Mittelung zu unterziehen, bevor die Berechnung der Ableitung erfolgt, um Fehlauslösungen zu vermeiden. Hierzu wird eine geeignete Anzahl von Messwerten aufsummiert und der Mittelwert durch Division mit der Anzahl der Messwerte in bekannter Weise berechnet. Sodann erfolgt vorteilhafterweise die Bestimmung eines zweiten Mittelwertes in identischer Weise. Die Berechnung der ersten Ableitung erfolgt nun wie oben beschrieben unter Verwendung der Mittelwerte, wobei das anzusetzende Zeitintervall dem Zeitraum entspricht, der für die Bestimmung eines Mittelwertes erforderlich war.

Es ist ein weiteres vorteilhaftes Merkmal der Vorrichtung, dass auch nach der Reduzierung der Ausgangsleistung weiterhin die Messung der ersten Ableitung des Widerstandes nach der Zeit in der oben beschriebenen Art und Weise erfolgt. Bereits kurz nachdem die Leistung reduziert wird, sammelt sich wieder Gewebewasser in den zuvor ausgetrockneten Bereichen, so dass der Widerstand wieder sinkt, bis ein konstanter Wert in der Nähe des ursprünglichen Ausgangswertes erreicht wird. Dieser Zeitpunkt ist **dadurch gekennzeichnet, dass** die erste Ableitung des zeitlichen Widerstandsverlaufes zunächst negativ ist und sich dem Wert null nähert. Damit ist der optimale Zeitpunkt detektiert, um die Ausgangsleistung wieder auf seinen ursprünglichen Wert einzustellen.

Dies wird vorzugsweise dadurch erreicht, dass die Messvorrichtung ein Signal an die Kontrolleinrichtung sendet, wenn die erste Ableitung des Widerstandsverlaufes negativ ist und sich in einem kleinen Intervall nahe null befindet. Ein typisches Intervall ist -3 Ohm/s bis 0 Ohm/s. Entsprechend ist die Mess- oder Rechenvorrichtung in Verbindung mit der Kontrolleinheit vorzugsweise dazu ausgebildet darauf anzusprechen, wenn die erste zeitliche Ableitung des Impedanz- oder ohmschen Widerstandsverlaufes einen Wert im Bereich von -10 Ohm/Sekunde bis -0,1 Ohm/Sekunde überschreitet. Diese Werte können in Abhängigkeit des zu behandelnden Gewebes einstellbar sein. Daraufhin regelt die Kontrolleinrichtung die Ausgangsleistung des Hochfrequenzstromes wieder auf den ursprünglichen Ausgangswert, wobei in der bevorzugten Ausführungsform der Ausgangswert über einen kurzen Zeitraum von typischerweise einigen Zehntelsekunden erhöht wird, um unerwünschte plötzliche Vaporisationseffekte zu vermeiden.

Die Mess- und Rechenvorrichtung kann entweder Teil der Kontrolleinrichtung sein oder außerhalb der Kontrolleinrichtung angeordnet und mit der Kontrolleinrichtung (Kontroller) verbunden sein. Die Mess- und Rechenvorrichtung kann außerdem in zwei separaten Einheiten verwirklicht sein, nämlich einer Messvorrichtung, der die Rechenvorrichtung nachgeschaltet ist oder einer integrierten Mess- und Rechenvorrichtung.

Bevorzugt ist eine Variante, bei der der Hochfrequenzgenerator, die Mess- und Rechenvorrichtung und die Kontrolleinrichtung in einem gemeinsamen Gehäuse untergebracht sind.

In einer bevorzugten Ausführungsform wird nach dem Reduzieren der Ausgangsleistung bei einsetzender Austrocknung nicht sofort die Ableitung des Widerstandsverlaufes weitergemessen bzw. ausgewertet, sondern zunächst eine Zwangswartezeit (Mindestzeit) mit reduzierter Ausgangsleistung im Bereich 1 Sekunde bis 10 Sekunden eingehalten. Dies ist vorteilhaft, da nach dem Reduzieren der Ausgangsleistung zunächst auch ein Plateau des Widerstandswertes gemessen wird, weil die Rückdiffusion von Wasser einige Zeit benötigt, um den Widerstand messbar wieder absinken zu lassen. Dadurch bildet sich für einen kurzen Zeitraum ein konstanter Widerstandswert auf hohem Niveau aus, der jedoch nicht mit dem konstanten Wert nach Rückdiffusion des Gewebewassers zu verwechseln ist. Dennoch könnte dieses Widerstandsplateau eine fehlerhafte Erhöhung der Ausgangsleistung auslösen, wenn nicht die Mindestwartezeit eingehalten wird, nach der sich in jedem Fall ein negativer Gradient des Widerstandsverlaufes einstellt. Nach Ablauf der Zwangswartezeit (Mindestzeit) zieht die Kontrolleinrichtung wieder die erste zeitliche Ableitung des Impedanzverlaufs für die weitere Steuerung der Ausgangsleistung des Hochfrequenzgenerators heran.

In einer Ausführungsvariante der Erfindung werden Elektrodennadeln mit einer offenen Spülung eingesetzt. Besonderes Merkmal hierbei ist, dass eine Pumpe, die den Flüssigkeitsstrom in das Gewebe vermittelt, zunächst nicht in Betrieb ist, d. h. der Applikator zunächst ohne offene Spülung arbeitet. Damit entfällt das Risiko der zuvor beschriebenen Zellverschleppung. Sobald die Vorrichtung nun aufgrund des Detektierens einer beginnenden Austrocknung die Ausgangsleistung des Hochfrequenzgenerators reduziert, wird von der Kontrolleinrichtung ein elektrisches Signal über einen Steueranschluss an die Pumpe übermittelt, einen kleinen Flüssigkeitsbolus in das ausgetrocknete Gewebe abzugeben. Dies bringt zwei Vorteile mit sich: zum einen kann die Flüssigkeitsmenge voreingestellt so bemessen werden, dass lediglich die Flüssigkeitsmenge injiziert wird, welche zuvor durch Austrocknung verloren gegangen ist. Damit kann es nicht zu einer flüssigkeitsvermittelten Zellverschleppung kommen. Zum zweiten wird der Prozess der Rehydrierung dadurch erheblich beschleunigt, weil nicht auf das natürliche Rückdiffundieren von Gewebewasser gewartet werden muss, so dass die Zeit bis zum Wiedereinsetzen der ursprünglichen Ausgangsleistung signifikant verkürzt wird.

In einer weiteren Ausführungsform ist die Menge des applizierten Flüssigkeitsbolus nicht voreingestellt. Vielmehr wird wie in der vorbeschriebenen Ausführungsvariante mittels eines Startsignals durch die Kontrolleinrichtung die Pumpe gestartet, wenn die Gewebeaustrocknung bevorsteht. Hierbei wird lediglich ein minimaler Flüssigkeitsstrom appliziert, der nicht sofort zu einer übermäßigen Flüssigkeitsansammlung führt. Die Applikation von Flüssigkeit erfolgt nun solange, bis die Kontrolleinrichtung erfindungsgemäß eine ausreichende Rehydrierung des Gewebes erfasst (über die erste Ableitung des zeitlichen Widerstandverlaufes) und das Signal zum Wiedereinsetzen der ursprünglichen Ausgangsleistung abgibt. Zu diesem Zeitpunkt wird ein Stopsignal an die Pumpe übermittelt, um den Flüssigkeitstransport zu beenden. Dieses Verfahren hat den Vorteil, dass durch die zusätzlich eingebrachte Flüssigkeit die Wartezeit bis zum Wiedereinsetzen der vollen Leistung verkürzt wird und gleichzeitig die Flüssigkeitsmenge sehr genau dem tatsächlichen Bedarf angepasst ist. Ist nämlich das Gewebevolumen wieder vollständig rehydriert, nimmt der Gewebewiderstand einen konstanten Verlauf und die Pumpe schaltet durch ein Stopsignal der Kontrolleinrichtung ab. Somit ist das Einbringen von zu viel Flüssigkeit verhindert und die Gefahr der Zellverschleppung nicht gegeben.

Zur Umsetzung dieser Verfahren weist die Hochfrequenz-Applikationsvorrichtung einen Steueranschluss für eine mit den in das Körpergewebe eingeführten Elektroden verbundene externe Pumpe auf, an den vorzugsweise eine Pumpe angeschlossen ist, die ausgebildet ist Flüssigkeit in zu behandelndes Körpergewebe zu fördern. Die Kontrolleinrichtung ist ausgebildet, ein Einschalten der Pumpe durch Abgabe eines Startsignals über den Steueranschluss zu veranlassen, wenn die Kontrolleinrichtung eine drohende Gewebeaustrocknung detektiert.

Dabei ist die Kontrolleinrichtung vorzugsweise ausgebildet, die externe Pumpe derart anzusteuern, dass ausschließlich durch solche Elektroden Flüssigkeit in das Behandlungsvolumen gefördert wird, zwischen denen die Kontrolleinrichtung eine drohende Gewebeaustrocknung detektiert hat.

Auch erfolgt das Einschalten der Pumpe und die Reduzierung der Ausgangsleistung - veranlasst durch die Kontrolleinheit - vorzugsweise gleichzeitig.

Die Pumpe ist vorzugsweise ausgebildet mit konstanter Fördermenge zu arbeiten. Erfasst die Hochfrequenz-Applikationsvorrichtung eine ausreichende Rehydrierung, schaltet die Kontrolleinrichtung die externe Pumpe in einer bevorzugten Ausführungsvariante wieder ab. Dies geschieht beispielsweise durch Abgabe eines Stopsignals über den Steueranschluss. Das Deaktivieren der Pumpe und das Zurückstellen der Ausgangsleistung auf die ursprüngliche Leistung erfolgt vorzugsweise zum gleichen Zeitpunkt.

Die verwendete Flüssigkeit kann sterile physiologische Kochsalzlösung oder steriles Wasser sein.

Der Vorteil der vorliegenden Erfindung ist neben der wesentlich einfacheren und sichereren Handhabung vor allem die Wirkung, dass durch die erfindungsgemäße Vorrichtung zu jedem Zeitpunkt der Therapie und in einem sehr weiten Bereich möglicher biologischer und physikalischer Parameter immer automatisch die maximal mögliche mittlere Leistung appliziert wird. Damit ist sichergestellt, dass in jedem Fall mit der maximalen Effizienz gearbeitet wird, d. h. dass für eine gegebene einzubringende Energiemenge immer nur die kürzest mögliche Zeit benötigt wird.

Vorkenntnisse sind für den Anwender der erfindungsgemäßen Hochfrequenzstrom-Applikationsvorrichtung nicht erforderlich. Einzige Voraussetzung ist, dass eine ausreichende Sollleistung voreingestellt wird. Hier hat sich in der Praxis bewährt, eine Leistungsvoreinstellung zu wählen, die nur von der Summe der Oberflächen oder Längen aller in das Körpergewebe eingebrachten Elektroden abhängt (größere Elektrodenfläche bedeutet größere Leistungseinstellung, z. B. 0,2 Watt/mm² oder 1 Watt/mm²). Dabei ist die Sollleistung etwas höher anzusetzen, als bei der ungünstigsten Kombination biologischer und physikalischer Parameter maximal erforderlich wäre. Damit ist sichergestellt, dass die beschriebene Regelfunktion der Vorrichtung immer im Verlauf einer Therapie einsetzt und die mittlere Leistung so an den optimalen Wert heranführen kann.

Im folgenden wird die Erfindung anhand der gezeigten Figuren näher erläutert. Von den Figuren zeigen:
- Figur 1 a: ein Blockdiagramm der erfindungsgemäßen Hochfrequenzapplikationsvorrichtung zur thermischen Verödung von Körpergewebe;
- Figur: 1 b ein Blockdiagramm der erfindungsgemäßen Hochfrequenzapplikationsvorrichtung zur thermischen Verödung von Körpergewebe mit einer externen Pumpe;
- Figur 1c: ein Blockdiagramm der erfindungsgemäßen Hochfrequenzapplikationsvorrichtung zur thermischen Verödung von Körpergewebe, mit einem unabhängigen Stromkreis zur Ermittlung der Impedanz oder des ohmschem Widerstands erforderlichen Parameter;
- Figur 2: ein Diagramm zur Darstellung des Widerstandsverlaufes zwischen den Elektroden eines Applikators in Abhängigkeit von der Applikationszeit während der Aktivierung des therapeutischen Hochfrequenzstromes im Körpergewebe;
- Figur 3: eine beispielhafte schematische Schnittdarstellung einer bipolaren Anordnung mit zwei Elektroden auf einem Schaft während der Aktivierung des therapeutischen Hochfrequenzstromes im Körpergewebe;
- Figur 4a: eine beispielhafte schematische Schnittdarstellung einer bipolaren Anordnung mit zwei Elektroden auf einem Schaft während der Inaktivierung (Rückdiffusion des Gewebewassers) des therapeutischen Hochfrequenzstromes im Körpergewebe;
- Figur 4b: eine beispielhafte schematische Schnittdarstellung einer bipolaren Anordnung mit zwei offen gespülten Elektroden auf einem Schaft während der Inaktivierung (Rückdiffusion des Gewebewassers und Einpumpen von Flüssigkeit) des therapeutischen Hochfrequenzstromes im Körpergewebe;
- Figur 5: Diagramme mit Darstellung der Ausgangsleistung, des Widerstandsverlaufes und der ersten Ableitung des Widerstandsverlaufes nach der Zeit zwischen zwei Elektroden in Abhängigkeit von der Applikati- onszeit während der Aktivierung des therapeutischen Hochfrequenzstromes im Körpergewebe; und
- Figur 6a: ein Flussdiagramm der erfindungsgemäßen Hochfrequenzapplikationsvorrichtung zur thermischen Verödung von Körpergewebe.
- Figur 6b: ein Flussdiagramm einer Ausführungsform der erfindungsgemäßen Hochfrequenzapplikationsvorrichtung zur thermischen Verödung von Körpergewebe mit externer Pumpe und mit einem voreinstellbaren applizierten Flüssigkeitsbolus.
- Figur 6c: ein Flussdiagramm einer Ausführungsform der erfindungsgemäßen Hochfrequenzapplikationsvorrichtung zur thermischen Verödung von Körpergewebe mit externer Pumpe ohne voreingestellte Menge des applizierten Flüssigkeitsbolus.
- Figur 7a: eine beispielhafte Darstellung eines Applikators mit einer bipolaren Anordnung von zwei Elektroden auf einem Schaft;
- Figur 7b: eine beispielhafte Darstellung mehrerer Applikatoren in einer multipolaren Anordnung mit jeweils zwei Elektroden auf einem Schaft pro Applikator;

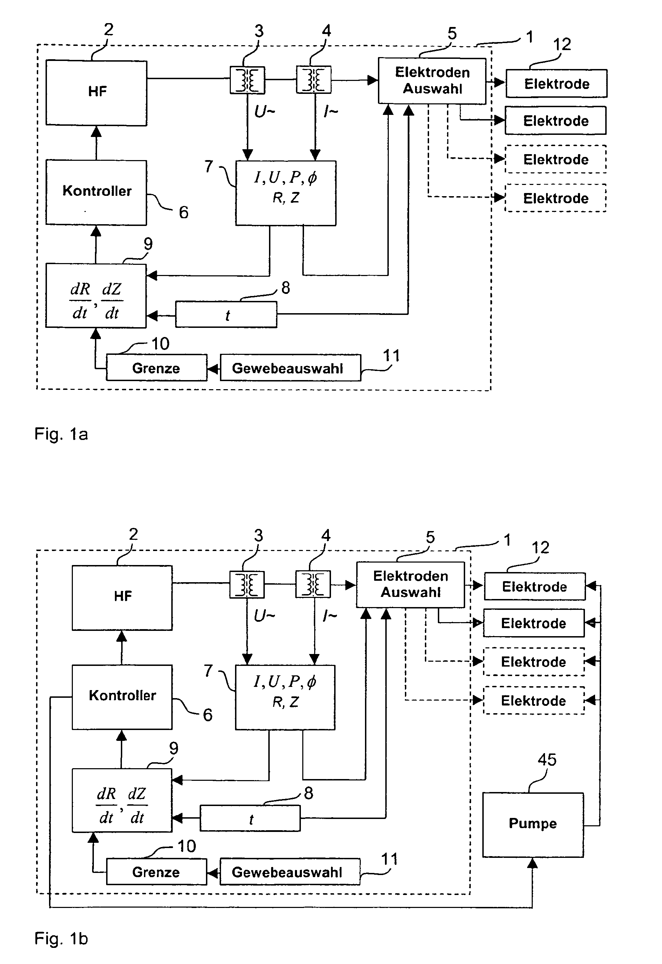

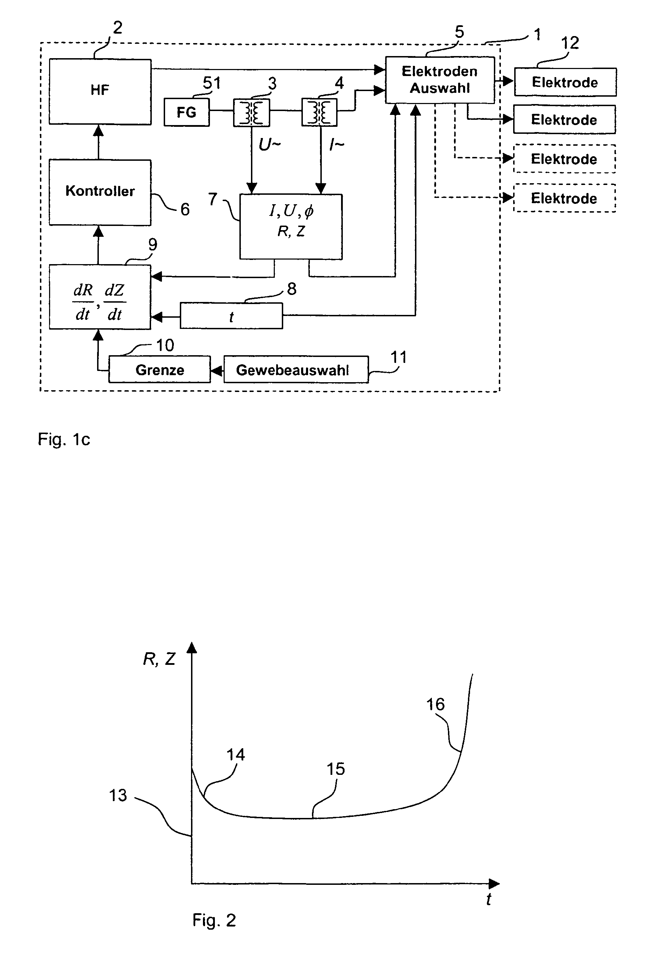

Fig. 1 a zeigt die Hochfrequenzapplikationsvorrichtung 1 zur thermischen Verödung von Körpergewebe, mit mindestens zwei Elektroden 12 die in Kontakt mit dem Körpergewebe stehen. Die Hochfrequenzapplikationsvorrichtung 1 ist bevorzugt in einem Gerät untergebracht. Die Hochfrequenzapplikationsvorrichtung 1 besteht aus einen Hochfrequenzgenerator 2 zum Erzeugen einer Hochfrequenzspannung, sowie einer Messvorrichtung 7 zum bestimmen der Impedanz bzw. des ohmschen Widerstands zwischen den Elektroden 12.

In einer bevorzugten Ausführungsform erfolgt die Bestimmung von Impedanz bzw. ohmschem Widerstand in der Messvorrichtung 7 mittels eines Hochfrequenzübertragers für die Spannung 3 und für den Strom 4 in bekannter Weise aus einer Kombination der Parameter Stromstärke, Spannung, Wirkleistung und Phasenverhalten des therapeutischen Hochfrequenzstromes.

Wesentliches Merkmal der Vorrichtung ist es nun, dass zur Detektion der Gewebeaustrocknung nicht der Absolutwert der Impedanz oder des ohmschen Widerstandes gemessen wird, sondern sein zeitliches Änderungsverhalten (Zeitmessung mittels eines Zeitgebers 8) durch die Auswertung der ersten Ableitung des Widerstandes in einer Rechenvorrichtung 9 ermittelt wird.

In einer bevorzugten Ausführungsform liefert die Messvorrichtung 7 in kurzen Zeitabständen von einigen Millisekunden jeweils ein neues Ergebnis der Widerstandsmessung. Von diesem Ergebnis wird z. B. durch einen Mikroprozessor das Ergebnis der vorhergehenden Messung subtrahiert und durch die Länge des Zeitintervalls zwischen beiden Messvorgängen geteilt, wobei die Länge des Zeitintervalls durch einen Zeitgeber 8 vorgegeben wird. Ist das Ergebnis positiv und liegt der Wert oberhalb eines vorgegebenen Grenzwertes 10 (oberer Schwellwert), welcher optional vor der Therapie mittels einer Gewebeauswahl-Vorrichtung 11 festgelegt werden kann, und der typischerweise im Bereich 5 Ohm/s bis 50 Ohm/s liegen sollte, liefert die Rechenvorrichtung 9 ein Signal an die Kontrolleinrichtung 6, dass die elektrodennahe Austrocknung unmittelbar bevorsteht. Die Kontrolleinrichtung reduziert daraufhin die Ausgangsleistung des Hochfrequenzgenerators 2 signifikant, um der Austrocknung entgegen zu wirken.

In einer bevorzugten Ausführungsform enthält die Hochfrequenzapplikationsvorrichtung 1 eine Elektrodenauswahl-Steuereinheit 5, wenn mehr als zwei Elektroden in einer multipolaren Arbeitsweise betrieben werden sollen, wobei die Elektroden-Steuereinheit 5 ebenfalls die Parameter Impedanz oder ohmscher Widerstand sowie Zeit verwendet, um die Elektroden geeignet anzusteuern.

Fig. 1b zeigt eine weitere Ausführungsform der in Fig. 1 a beschriebenen Hochfrequenzapplikationsvorrichtung 1 mit einer externen Pumpe 45. Sobald die Hochfrequenzapplikationsvorrichtung 1 eine beginnende Austrocknung detektiert, wird die Ausgangsleistung des Hochfrequenzgenerators 2 reduziert und die Kontrolleinrichtung 6 übermittelt ein elektrisches Signal an die Pumpe, einen voreingestellten kleinen Flüssigkeitsbolus an die Elektroden 12 und somit in das ausgetrocknete Gewebe abzugeben.

In einer bevorzugten Ausführungsform wird von der durch die Kontrolleinrichtung 6 mittels Signal gestarteten Pumpe 45 solange ein Flüssigkeitsstrom appliziert, bis die Kontrolleinrichtung 6 eine ausreichende Rehydrierung des Gewebes erfasst und ein Signal zum Wiedereinsetzen der ursprünglichen Ausgangsleistung abgibt. Zu diesem Zeitpunkt wird ein weiteres Signal an die Pumpe 45 übermittelt, den Flüssigkeitstransport zu beenden.

Fig. 1c zeigt eine alternative Ausführungsform bei der der Widerstand auch unabhängig vom therapeutischen Hochfrequenzstrom mit Hilfe eines separaten Frequenzgenerators 51 ermittelt wird. Bei dieser Anordnung ist es nicht erforderlich, während der Rehydrierungsphase den therapeutischen Hochfrequenzstrom mit kleiner Leistung aufrecht zu erhalten, um Messwerte der Impedanz oder des ohmschen Widerstandes zu ermitteln. Daher kann in dieser Anordnung bei beginnender Austrocknung die Ausgangsleistung des Hochfrequenzgenerators auch ganz abgeschaltet werden. Messfrequenz und Messstrom sind vom therapeutischen Hochfrequenzstrom unabhängig und können z. B. so gewählt werden, dass sowohl Gewebeänderungen optimal erfasst werden als auch keine signifikante Gewebeerwärmung beim Messvorgang auftritt.

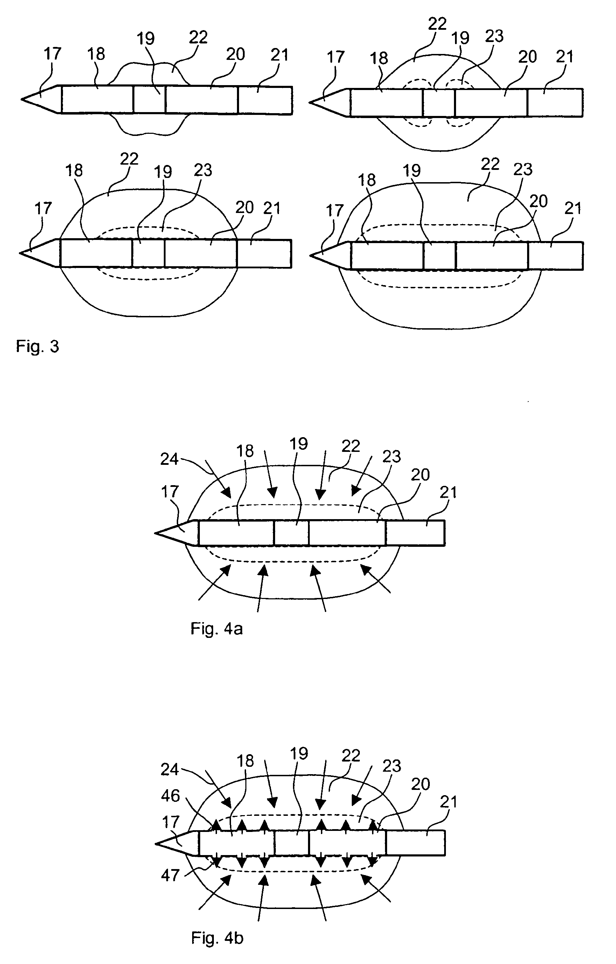

Gemäß dem Diagramm 13 aus Fig. 2 sinken die Impedanz und der ohmsche Gewebewiderstand 14 zunächst signifikant unter den Ausgangswert, nach dem Aktivieren der Hochfrequenzspannung an zwei Elektroden die sich im elektrischen Kontakt mit dem Körpergewebe befinden. Dies ist bedingt durch die beginnende Gewebeerwärmung und die damit verbundene bessere Beweglichkeit der den elektrischen Stromfluss vermittelnden Ionen im Körpergewebe (z. B. Na⁺, Cl⁻). Bei einem Anstieg der Temperatur über 50 °C, kommt es zu einer massiven Denaturierung der körpereigenen Proteine (Koagulation) 22 und in der Folge zum Absterben der betroffenen Gewebeareale (siehe Fig. 3). Es kommt zu einer allmählichen Ausbreitung der Koagulation 22. Diese zeichnet sich durch einen relativ konstanten Verlauf 15 der Impedanz bzw. des ohmschen Widerstandes aus.

Fig. 3 zeigt beispielhaft die kontinuierliche Ausbreitung der oben genannten Koagulationszone 22 im Körpergewebe im Bereich der auf einen Schaft 21 axial angeordneten Elektroden 18, 20 (die durch einen Isolator 19 elektrisch voneinander isoliert sind) bei zeitlich fortschreitender Applizierung des Hochfrequenzstromes. Der Koagulierungsprozess beginnt in dem angrenzenden Körpergewebe, welches im Bereich der einander benachbarten Zonen der Elektroden 18, 20 angesiedelt ist. Das Koagulierungsareal breitet sich dann nach vorn zur freien Spitze 17 der Elektrodenanordnung und nach proximal zum proximalen Ende der Elektrode 20 aus.

Mit zunehmender Applikationszeit wird im Körpergewebe eine Temperatur von ca. 100 °C erreicht und das Gewebewasser beginnt in die Dampfphase überzutreten. Aufgrund der mit dem Übertritt des Wassers von der Flüssigphase in die Dampfphase verbundenen Ausdehnung entfernt sich der Dampf durch Gewebespalten von den elektrodennahen Bereichen, in denen typischerweise die höchsten Temperaturen zu verzeichnen sind. Diese sogenannte Vaporisation geht somit einher mit einer Verringerung des Wassergehaltes in jenen elektrodennahen Bereichen, so dass es allmählich zu einer Austrocknungszone 23 im Gewebe kommt, welche sich schließlich über die Länge beider Elektroden 18, 20 ausdehnt. Zunächst reicht das restliche in den elektrodennahen Bereichen verbliebene Zellwasser noch aus, um die Beweglichkeit der den Stromfluss vermittelnden Ionen zu gewährleisten, jedoch wird mit zunehmendem Austrocknungsgrad die Beweglichkeit der Ionen mehr und mehr eingeschränkt. Dies ist der Zeitpunkt, an dem die Austrocknungszone 23 die gesamte Fläche der Elektroden 18, 20 umschließt und der Gewebewiderstand 16 (gemäß Fig. 2) signifikant ansteigt.

Gemäß Fig. 4a kommt es nach Reduzierung der Ausgangsleistung zu einer Rückdiffusion des Gewebewassers 24 in den zuvor ausgetrockneten Bereich 23, so dass der Widerstand wieder sinkt, bis ein konstanter Wert in der Nähe des ursprünglichen Plateauwertes 15 (gemäß Fig. 2) erreicht wird.

Bei der in Fig. 4b dargestellten bevorzugten Ausführungsform der Erfindung werden Elektroden 18 mit einer offenen Spülung eingesetzt. Nach der Reduzierung der Ausgangsleistung wird Flüssigkeit 47 durch die Öffnungen 46 der Elektroden 18 in den zuvor ausgetrockneten Bereich 23 abgegeben. Es wird in etwa die Flüssigkeitsmenge abgegeben, welche zuvor durch Austrocknung verloren gegangen ist. Dadurch wird der Prozess der Rehydrierung des Gewebewassers 24 erheblich beschleunigt, weil nicht auf das natürliche Rückdiffundieren von Gewebewasser 24 gewartet werden muss, so dass der Widerstand wieder sinkt und die Zeit bis zum Wiedereinsetzen der ursprünglichen Ausgangsleistung signifikant verkürzt wird.

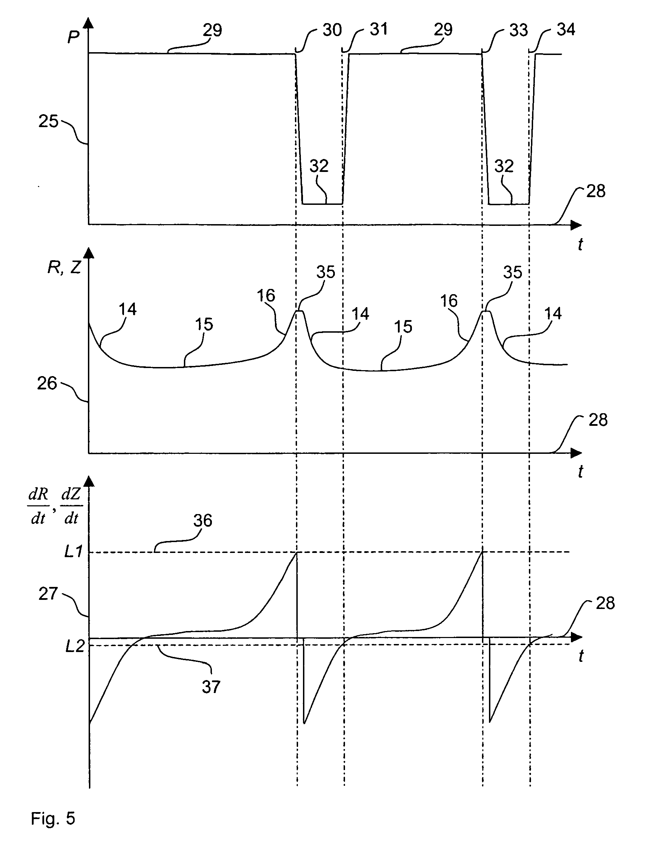

Fig. 5 zeigt den typischen zeitlichen Verlauf von Ausgangsleistung 25, Impedanz bzw. ohmschem Widerstand 26 und der zeitlichen Ableitung von Impedanz oder ohmschem Widerstand 27 während der Verödung von Körpergewebe mit der erfindungsgemäßen Hochfrequenzstromapplikationsvorrichtung.

Nach dem Aktivieren der Hochfrequenzspannung sinkt der Gewebewiderstand gemäß Diagramm 26 zunächst signifikant unter den Ausgangswert (14). Bei gleich bleibender Ausgangsleistung gemäß Diagramm 25 kommt es zunächst zu der in Fig. 2 beschriebenen Plateaubildung 15 mit einer folgenden stetigen Steigung des Widerstandes bis die Austrocknung einsetzt und der Widerstand exponentiell ansteigt (16).

Die erste Ableitung des zeitlichen Widerstandsverlaufes 26 zeigt zu den korrespondierenden Zeitpunkten zunächst negative Werte gemäß Diagramm 27, die sich dem Wert null annähern, um dann in den positiven Bereich überzugehen. Ist das Ergebnis positiv und liegt der Wert oberhalb eines vorgegebenen Grenzwertes 36 (oberer Schwellwert), welcher vor der Therapie festgelegt wird, liefert die Rechenvorrichtung 9 zum Zeitpunkt 30 ein Signal an die Kontrolleinrichtung 6 (siehe Fig. 1 a), dass die elektrodennahe Austrocknung unmittelbar bevorsteht. Die Ausgangsleistung gemäß Diagramm 25 wird sofort reduziert, um der Austrocknung entgegen zu wirken. Der Widerstand bleibt kurzzeitig auf dem Niveau 35 nach der Reduzierung der Ausgangsleistung 32, weil die Rückdiffusion von Wasser einige Zeit benötigt, um den Widerstand messbar wieder absinken zu lassen. Bereits kurz nachdem die Leistung reduziert wird 32, sammelt sich wieder Gewebewasser in den zuvor ausgetrockneten Bereichen, so dass der Widerstand wieder sinkt 14, bis ein konstanter Wert in der Nähe des ursprünglichen Plateauwertes 15 erreicht wird. Dies ist **dadurch gekennzeichnet, dass** die erste Ableitung des zeitlichen Widerstandsverlaufes zunächst negativ ist und sich dann einem Grenzwert 37 (unterer Schwellwert) nahe null nähert. Ein typischer Wert ist -3 Ohm/s bis 0 Ohm/s. Damit ist der optimale Zeitpunkt 31 detektiert, um die Ausgangsleistung wieder auf seinen ursprünglichen Wert einzustellen. Dies wird dadurch erreicht, dass die Rechenvorrichtung 9 ein Signal an die Kontrolleinrichtung 6 sendet (siehe Fig. 1 a). Daraufhin regelt die Kontrolleinrichtung die Ausgangsleistung des Hochfrequenzstromes wieder auf den ursprünglichen Ausgangswert 29, wobei in der bevorzugten Ausführungsform der Ausgangswert über einen kurzen Zeitraum von typischerweise einigen Zehntelsekunden erhöht wird, um unerwünschte plötzliche Vaporisationseffekte zu vermeiden. Der beschriebene Vorgang kann sich dann in unregelmäßigen Abständen beliebig oft wiederholen (33, 34), bis die Therapie manuell abgebrochen wird.

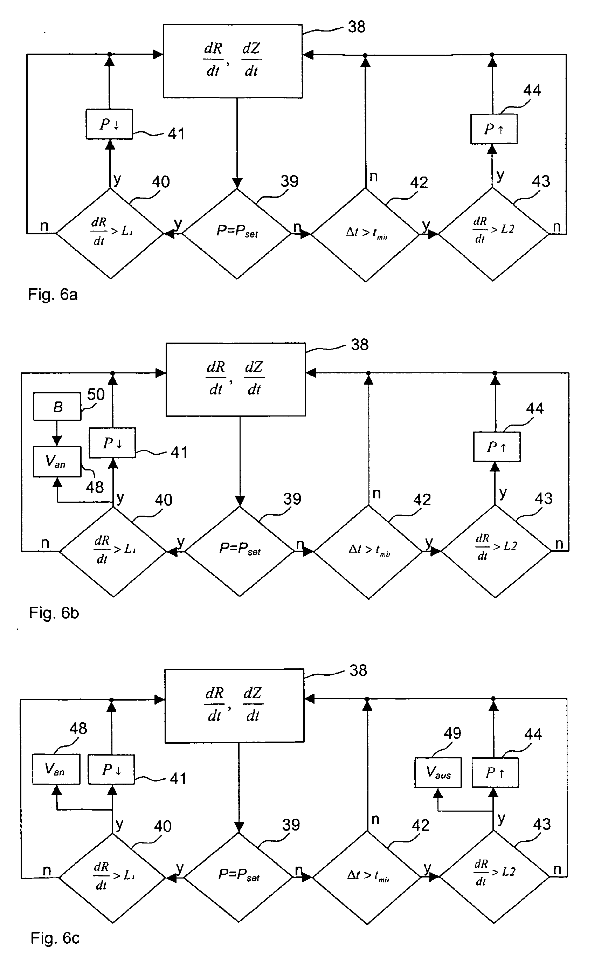

In Fig. 6a ist die Funktionsweise der Rechenvorrichtung 9 aus Fig. 1 a als Flussdiagramm dargestellt. Nach Bestimmung der ersten Ableitung des Widerstandes nach der Zeit 38, wird die tatsächliche Ausgangsleistung mit der eingestellten Ausgangsleistung verglichen (39). Befindet sich die Ausgangsleistung bei der Sollleistung 29 wird in einem weiteren Schritt 40 überprüft, ob der Wert der ersten Ableitung des Widerstandes nach der Zeit oberhalb des vorgegebenen Grenzwertes 36 liegt. Wenn ja, erfolgt der Befehl 41, die Ausgangsleistung zu reduzieren (32). Ist die erste Ableitung des Widerstandes nach der Zeit dagegen noch unterhalb des vorgegebenen Grenzwertes 36 (40), erfolgt die nächste Bestimmung der ersten Ableitung des Widerstandes nach der Zeit 38 und der Zyklus wird von neuem durchlaufen.

Wird in 39 dagegen festgestellt, dass die tatsächliche Ausgangsleistung sich bereits auf dem erniedrigten Niveau befindet (32), erfolgt zunächst die Überprüfung 42, ob die Mindestwartezeit vor einem möglichen Zurücksetzen der Ausgangsleistung auf den ursprünglichen Sollwert 29 eingehalten wurde. Wurde die Mindestwartezeit 42 eingehalten, dann wird die Ableitung des Widerstandsverlaufes im Schritt 43 mit dem voreingestellten Grenzwert 37 verglichen. Sollte die Ableitung größer sein, als dieser Grenzwert 37, erfolgt der Befehl 44, die Ausgangsleistung wieder auf den Sollwert 29 einzustellen. Wurde die Mindestwartezeit 42 noch nicht eingehalten oder ist die zeitliche Ableitung des Widerstandsverlaufes noch kleiner als der Grenzwert 37, erfolgt die nächste Bestimmung der ersten Ableitung des Widerstandes nach der Zeit 38 und der Zyklus wird von neuem durchlaufen.

Es erwies sich als vorteilhaft eine Mindestwartezeit 42 abzufragen, da nach dem Reduzieren der Ausgangsleistung 32 zunächst auch das Plateau des Widerstandswertes gemessen wird, weil die Rückdiffusion von Wasser einige Zeit benötigt, um den Widerstand messbar wieder absinken zu lassen. Dadurch bildet sich für einen kurzen Zeitraum ein konstanter Widerstandswert auf hohem Niveau 35 aus, der jedoch nicht mit dem konstanten Wert nach Rückdiffusion des Gewebewassers zu verwechseln ist. Dennoch könnte dieses Widerstandsplateau eine fehlerhafte Erhöhung der Ausgangsleistung auslösen, wenn nicht die Mindestwartezeit 42 vorzugsweise im Bereich 1 Sekunde bis 10 Sekunden eingehalten wird, nach der sich in jedem Fall ein negativer Gradient des Widerstandsverlaufes einstellt.

Fig. 6b zeigt eine bevorzugte Ausführungsform eines Flussdiagramms der in Fig. 6a beschriebenen Funktionsweise der Rechenvorrichtung 9 aus Fig. 1 a. Sobald eine beginnende Austrocknung des Gewebes detektiert wird, d. h. der Wert der ersten Ableitung des Widerstandes nach der Zeit liegt oberhalb des vorgegebenen Grenzwertes 36, wird die Ausgangsleistung reduziert (41). Gleichzeitig wird ein kleiner Flüssigkeitsbolus abgegeben (48), der über eine Einrichtung 50 voreingestellt werden kann.

Fig. 6c zeigt eine weitere bevorzugte Ausführungsform eines erweiterten Flussdiagramms der in Fig. 6a beschriebenen Funktionsweise der Rechenvorrichtung 9 aus Fig. 1 a. Wird eine beginnende Austrocknung des Gewebes detektiert, d. h. der Wert der ersten Ableitung des Widerstandes nach der Zeit liegt oberhalb des vorgegebenen Grenzwertes 36, dann wird die Ausgangsleistung reduziert (41). Gleichzeitig wird ein minimaler Flüssigkeitsstrom gestartet (48). Dieser erfolgt solange, bis die Ableitung des Widerstandsverlaufes im Schritt 43 größer ist als der voreingestellte Grenzwert 37 und die Ausgangsleistung (44) wieder auf den Sollwert 29 eingestellt wird. Der Flüssigkeitsstrom wird angehalten (49).

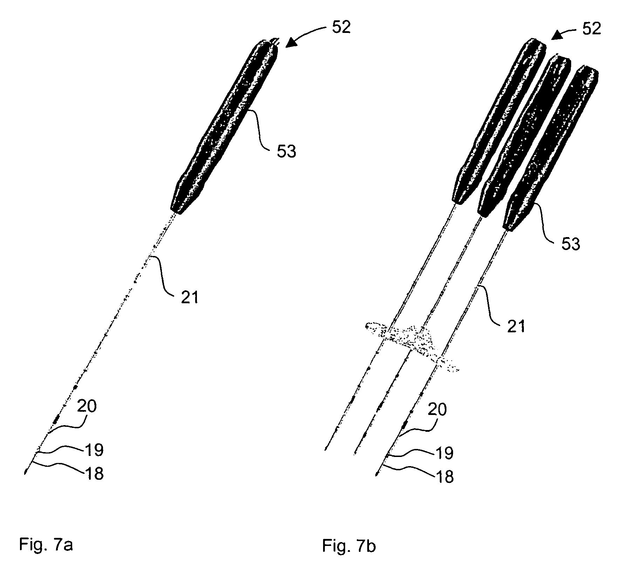

Fig. 7a zeigt eine bevorzugte Ausführungsform eines Applikators mit einer bipolaren Anordnung von zwei Elektroden auf einem Schaft. Der Applikator 52 besteht aus einem Handgriff 53 und einem Schaft 21, der mit dem Handgriff verbunden ist und zwei axial zueinander angeordnete Elektroden 18, 20 (die durch einen Isolator 19 elektrisch voneinander isoliert sind) aufweist.

Fig. 7b zeigt eine weitere Ausführungsform mehrerer Applikatoren in einer multipolaren Anordnung mit jeweils zwei Elektroden auf einem Schaft pro Applikator. Die Applikatoren 52 bestehen jeweils aus einem Handgriff 53 und einem Schaft 21, der mit dem Handgriff verbunden ist und zwei axial zueinander angeordnete Elektroden 18, 20 (die durch einen Isolator 19 elektrisch voneinander isoliert sind) aufweist.

## Patentansprüche

1. Hochfrequenzstrom-Applikationsvorrichtung zur thermischen Verödung von Körpergewebe, mit einem Hochfrequenzgenerator, an den mindestens zwei Elektroden angeschlossen sind, die zum Kontakt mit Körpergewebe ausgebildet sind, sowie mit einer Mess- und Rechenvorrichtung, die ausgebildet ist, aus mindestens zwei der Parameter Stromstärke, Spannung, Phasenbeziehung und Wirkleistung die Impedanz oder den ohmschen Widerstand zwischen den zwei Elektroden, zwischen denen jeweils ein therapeutischer Hochfrequenzstrom fließt oder mittels eines vom therapeutischen Stromkreis unabhängigen Messkreises zu ermitteln, und einer mit der Mess- und Rechenvorrichtung verbundenen Kontrolleinrichtung zur Veränderung der Ausgangsleistung, wobei
- die Mess- und Rechenvorrichtung ausgebildet ist, während einer Hochfrequenzstromapplikation aus dem zeitlichen Verlauf der Impedanz oder des ohmschen Widerstandes die erste Ableitung zu bilden und ein vom Wert der ersten Ableitung abhängiges Signal abzugeben, und
- die Kontrolleinrichtung ausgebildet ist, die Ausgangsleistung signifikant zu reduzieren, wenn das Signal der Mess- und Rechenvorrichtung anzeigt, dass die zeitliche Ableitung der Impedanz oder des ohmschen Widerstandes eine positive Schwelle überschreitet, die für eine drohende Gewebeaustrocknung charakteristisch ist und
**dadurch gekennzeichnet, dass** die Kontrolleinrichtung ausgebildet ist,
- die Ausgangsleistung wieder auf die ursprüngliche Leistung zurückzustellen, wenn das Signal der Mess- und Rechenvorrichtung anzeigt, dass die erste Ableitung des zeitlichen Impedanz- oder ohmschen Widerstandsverlaufes eine negative Schwelle, die für eine ausreichende Rehydrierung des Körpergewebes charakteristisch ist, in Richtung positiver Werte überschreitet.

2. Hochfrequenzstrom-Applikationsvorrichtung nach Anspruch 1, **dadurch gekennzeichnet, dass** die Mess- und Rechenvorrichtung ausgebildet ist, die zur Ermittlung von Impedanz oder ohmschem Widerstand erforderlichen Parameter aus einem zwischen den Elektroden fließenden therapeutischen Hochfrequenzstrom abzuleiten.

3. Hochfrequenzstrom-Applikationsvorrichtung nach den Anspruch 2, **dadurch gekennzeichnet, dass** die Kontrolleinrichtung ausgebildet ist, die Ausgangsleistung auf einen Wert im Bereich 2 % bis 50 % der ursprünglichen Ausgangsleistung zu reduzieren, wenn dass Signal der Mess- und Rechenvorrichtung anzeigt, dass die erste Ableitung der Impedanz oder des ohmschen Widerstandes die positive Schwelle überschreitet.

4. Hochfrequenzstrom-Applikationsvorrichtung nach Anspruch 1, **dadurch gekennzeichnet, dass** die Mess- und Rechenvorrichtung ausgebildet ist, die zur Ermittlung von Impedanz oder ohmschem Widerstand erforderlichen Parameter allein mittels eines vom therapeutischen Stromkreis unabhängigen Messkreises abzuleiten.

5. Hochfrequenzstrom-Applikationsvorrichtung nach Anspruch 4, **dadurch gekennzeichnet, dass** die Kontrolleinrichtung ausgebildet ist, die Ausgangsleistung auf einen Wert im Bereich 0 % bis 50 % der ursprünglichen Ausgangsleistung zu reduzieren, wenn das Signal der Mess- und Rechenvorrichtung anzeigt, dass die erste Ableitung der Impedanz oder des ohmschen Widerstandes die positive Schwelle überschreitet.

6. Hochfrequenzstrom-Applikationsvorrichtung nach Anspruch 4, **dadurch gekennzeichnet, dass** der getrennte Messkreis bei einer anderen als der Arbeitsfrequenz der therapeutischen Hochfrequenzstromquelle arbeitet.

7. Hochfrequenzstrom-Applikationsvorrichtung nach Anspruch 1, **dadurch gekennzeichnet, dass** die Mess- und Rechenvorrichtung ausgebildet ist, die erste zeitliche Ableitung des Impedanz- oder ohmschen Widerstandsverlaufes aus zwei zeitlich aufeinander folgenden Messwerten der Impedanz oder des ohmschen Widerstandes zu bilden.

8. Hochfrequenzstrom-Applikationsvorrichtung nach Anspruch 1, **dadurch gekennzeichnet, dass** die Mess- und Rechenvorrichtung ausgebildet ist, einen Mittelwert einer Anzahl zeitlich aufeinander folgender Messwerte der Impedanz oder des ohmschen Widerstandes zu berechnen, und die erste zeitliche Ableitung des Impedanz- oder ohmschen Widerstandsverlaufes aus zwei zeitlich aufeinander folgenden Mittelwerten der Impedanz oder des ohmschen Widerstandes zu bilden.

9. Hochfrequenzstrom-Applikationsvorrichtung nach Anspruch 1, **dadurch gekennzeichnet, dass** die Kontrolleinrichtung ausgebildet ist, die Ausgangsleistung zu reduzieren, wenn das Signal der Mess- und Rechenvorrichtung anzeigt, dass die erste zeitliche Ableitung des Impedanz- oder ohmschen Widerstandsverlaufes einen Wert im Bereich 5 Ohm/Sekunde bis 50 Ohm/Sekunde überschreitet.

10. Hochfrequenzstrom-Applikationsvorrichtung nach Anspruch 1, **dadurch gekennzeichnet, dass** die Kontrolleinrichtung ausgebildet ist, die Ausgangsleistung über einen kurzen Zeitraum kontinuierlich zu reduzieren.

11. Hochfrequenzstrom-Applikationsvorrichtung nach Anspruch 10, **dadurch gekennzeichnet, dass** der Zeitraum zwischen 0,1 Sekunden und 5 Sekunden lang ist.

12. Hochfrequenzstrom-Applikationsvorrichtung nach Anspruch 1, **dadurch gekennzeichnet, dass** die Kontrolleinrichtung ausgebildet ist, die reduzierte Ausgangsleistung unabhängig vom zeitlichen Impedanz- oder Widerstandsverlauf für eine Mindestzeit beizubehalten.

13. Hochfrequenzstrom-Applikationsvorrichtung nach Anspruch 12, **dadurch gekennzeichnet, dass** die Mindestzeit zwischen 1 Sekunde und 10 Sekunden lang ist.

14. Hochfrequenzstrom-Applikationsvorrichtung nach Anspruch 13, **dadurch gekennzeichnet, dass** die Kontrolleinrichtung ausgebildet ist, nach Ablauf der Mindestzeit wieder die erste Ableitung des zeitlichen Impedanz- oder Widerstandsverlauf für die weitere Steuerung der Leistungsabgabe heranzuziehen.

15. Hochfrequenzstrom-Applikationsvorrichtung nach Anspruch 1 oder 14, **dadurch gekennzeichnet, dass** die Kontrolleinrichtung ausgebildet ist, die Ausgangsleistung wieder zu erhöhen, wenn das Signal der Mess- und Rechenvorrichtung anzeigt, dass die erste zeitliche Ableitung des Impedanz- oder ohmschen Widerstandsverlaufes einen Wert im Bereich minus 10 Ohm/Sekunde bis minus 0,1 Ohm/Sekunde überschreitet.

16. Hochfrequenzstrom-Applikationsvorrichtung nach Anspruch 9 oder 15, **dadurch gekennzeichnet, dass** für unterschiedliche Gewebearten unterschiedliche Schwellwerte wählbar sind.

17. Hochfrequenzstrom-Applikationsvorrichtung nach Anspruch 1, **dadurch gekennzeichnet, dass** die Kontrolleinrichtung ausgebildet ist, die Ausgangsleistung über einen kurzen Zeitraum kontinuierlich auf die ursprüngliche Leistung zurückzustellen.

18. Hochfrequenzstrom-Applikationsvorrichtung nach Anspruch 17, **dadurch gekennzeichnet, dass** der Zeitraum zwischen 0,1 Sekunden und 5 Sekunden lang ist.

19. Hochfrequenzstrom-Applikationsvorrichtung nach einem der vorgenannten Ansprüche, **dadurch gekennzeichnet, dass** der Hochfrequenzgenerator, die Mess- und Rechenvorrichtung und die Kontrolleinrichtung in einem gemeinsamen Gehäuse untergebracht sind.

20. Hochfrequenzstrom-Applikationsvorrichtung nach Anspruch 1, **dadurch gekennzeichnet, dass** die zwei Elektroden, zwischen denen die Impedanz- oder ohmsche Widerstandsmessung erfolgt, auf einem Schaft angeordnet sind, der in das Körpergewebe einzuführen ist.

21. Hochfrequenzstrom-Applikationsvorrichtung nach Anspruch 1, **dadurch gekennzeichnet, dass** die zwei Elektroden, zwischen denen die Impedanz- oder ohmsche Widerstandsmessung erfolgt, auf zwei unterschiedlichen Schäften angeordnet sind, die beide in das Körpergewebe einzuführen sind.

22. Hochfrequenzstrom-Applikationsvorrichtung nach Anspruch 1, **dadurch gekennzeichnet, dass** mindestens eine der zwei Elektroden, zwischen denen die Impedanz- oder ohmsche Widerstandsmessung erfolgt, aus mehreren getrennten Elementen gebildet ist, die entweder auf dem selben Schaft oder auf unterschiedlichen Schäften angeordnet sind und durch eine elektrisch leitende Verbindung auf demselben elektrischen Potenzial liegen.

23. Hochfrequenzstrom-Applikationsvorrichtung nach Anspruch 1, **dadurch gekennzeichnet, dass** eine der zwei Elektroden, zwischen denen die Impedanz- oder ohmsche Widerstandsmessung erfolgt, eine auf die Hautoberfläche aufzubringende Neutralelektrode ist.

24. Hochfrequenzstrom-Applikationsvorrichtung nach Anspruch 23, **dadurch gekennzeichnet, dass** die Neutralelektrode aus mehreren getrennten Elementen gebildet wird, die auf unterschiedliche Hautoberflächen aufzubringen sind und durch eine elektrisch leitende Verbindung auf demselben elektrischen Potenzial liegen.

25. Hochfrequenz-Applikationsvorrichtung nach einem der Ansprüche 1 bis 24, **dadurch gekennzeichnet, dass** die Hochfrequenz-Applikationsvorrichtung einen Steueranschluss für eine mit den in das Körpergewebe eingeführten Elektroden verbundene externe Pumpe aufweist.

26. Hochfrequenz-Applikationsvorrichtung nach Anspruch 25, **dadurch gekennzeichnet, dass** die Hochfrequenz-Applikationsvorrichtung mit einer Pumpe verbunden ist, die ausgebildet ist Flüssigkeit in zu behandelndes Körpergewebe zu fördern.

27. Hochfrequenz-Applikationsvorrichtung nach einem der Ansprüche 25 oder 26, **dadurch gekennzeichnet, dass** die Kontrolleinrichtung ausgebildet ist, ein Einschalten der Pumpe zu veranlassen, wenn die Kontrolleinrichtung eine drohende Gewebeaustrocknung detektiert.

28. Hochfrequenz-Applikationsvorrichtung nach Anspruch 27, **dadurch gekennzeichnet, dass** die Kontrolleinrichtung ausgebildet ist die externe Pumpe derart anzusteuern, dass ausschließlich durch solche Elektroden Flüssigkeit in das Behandlungsvolumen gefördert wird, zwischen denen die Kontrolleinrichtung eine drohende Gewebeaustrocknung detektiert hat.

29. Hochfrequenz-Applikationsvorrichtung nach Anspruch 27 oder 28, **dadurch gekennzeichnet, dass** die Kontrolleinrichtung ausgebildet ist das Einschalten der Pumpe und die Reduzierung der Ausgangsleistung gleichzeitig zu veranlassen.

30. Hochfrequenz-Applikationsvorrichtung nach Anspruch 25 bis 29, **dadurch gekennzeichnet, dass** die Kontrolleinrichtung ausgebildet ist die Pumpe derart anzusteuern, dass die Pumpe eine vorher definierte Flüssigkeitsmenge in das Behandlungsvolumen fördert, wobei die Flüssigkeitsmenge derart bemessen ist, dass sie in etwa der Menge zuvor verdampften Gewebewassers entspricht.

31. Hochfrequenz-Applikationsvorrichtung nach Anspruch 26, **dadurch gekennzeichnet, dass** die Pumpe ausgebildet ist mit konstanter Fördermenge zu arbeiten und die Kontrolleinrichtung ausgebildet ist, die externe Pumpe bei ausreichender Geweberehydrierung wieder zu deaktivieren.

32. Hochfrequenz-Applikationsvorrichtung nach Anspruch 31, **dadurch gekennzeichnet, dass** die Kontrolleinrichtung ausgebildet ist das Deaktivieren der Pumpe und das Zurückstellen der Ausgangsleistung auf die ursprüngliche Leistung zum gleichen Zeitpunkt zu bewirken.

33. Hochfrequenz-Applikationsvorrichtung nach Anspruch 26, **dadurch gekennzeichnet, dass** die Flüssigkeit sterile physiologische Kochsalzlösung ist.

34. Hochfrequenz-Applikationsvorrichtung nach Anspruch 26, **dadurch gekennzeichnet, dass** die Flüssigkeit steriles Wasser ist.

## Claims

1. High-frequency current application device for the thermal sclerosing of body tissue, having a high-frequency generator to which there are connected at least two electrodes which are designed for contact with body tissue, and having a measuring and calculating device which is designed to determine the impedance or ohmic resistance between the two electrodes, between which a therapeutic high-frequency current flows, from at least two of the parameters current strength, voltage, phase relation and effective power or by means of a measuring circuit which is independent of the therapeutic current circuit, and having a control device, connected to the measuring and calculating device, for changing the output power, wherein
- the measuring and calculating device is designed, during a high-frequency current application, to form the first derivative from the variation over time of the impedance or ohmic resistance and to deliver a signal dependent on the value of the first derivative, and
- the control device is designed to reduce the output power significantly when the signal of the measuring and calculating device indicates that the time derivative of the impedance or ohmic resistance exceeds a positive threshold which is characteristic of imminent tissue desiccation,
**characterised in that** the control device is designed to restore the output power to the original power again when the signal of the measuring and calculating device indicates that the first derivative of the variation over time of the impedance or ohmic resistance exceeds a negative threshold which is characteristic of adequate rehydration of the body tissue, in the direction of positive values.

2. High-frequency current application device according to claim 1, **characterised in that** the measuring and calculating device is designed to derive the parameters necessary for determining the impedance or ohmic resistance from a therapeutic high-frequency current flowing between the electrodes.

3. High-frequency current application device according to claim 2, **characterised in that** the control device is designed to reduce the output power to a value in the range of from 2% to 50% of the original output power when the signal of the measuring and calculating device indicates that the first derivative of the impedance or ohmic resistance exceeds the positive threshold.

4. High-frequency current application device according to claim 1, **characterised in that** the measuring and calculating device is designed to derive the parameters necessary for determining the impedance or ohmic resistance solely by means of a measuring circuit which is independent of the therapeutic current circuit.

5. High-frequency current application device according to claim 4, **characterised in that** the control device is designed to reduce the output power to a value in the range of from 0% to 50% of the original output power when the signal of the measuring and calculating device indicates that the first derivative of the impedance or ohmic resistance exceeds the positive threshold.

6. High-frequency current application device according to claim 4, **characterised in that** the separate measuring circuit operates at a frequency other than the working frequency of the therapeutic high-frequency current source.

7. High-frequency current application device according to claim 1, **characterised in that** the measuring and calculating device is designed to form the first time derivative of the variation of the impedance or ohmic resistance from two measured values of the impedance or ohmic resistance which are successive in terms of time.

8. High-frequency current application device according to claim 1, **characterised in that** the measuring and calculating device is designed to calculate a mean value of a number of measured values of the impedance or ohmic resistance which are successive in terms of time and to form the first time derivative of the variation of the impedance or ohmic resistance from two mean values of the impedance or ohmic resistance which are successive in terms of time.

9. High-frequency current application device according to claim 1, **characterised in that** the control device is designed to reduce the output power when the signal of the measuring and calculating device indicates that the first time derivative of the variation of the impedance or ohmic resistance exceeds a value in the range of from 5 ohms/second to 50 ohms/second.

10. High-frequency current application device according to claim 1, **characterised in that** the control device is designed to reduce the output power continuously over a short period of time.

11. High-frequency current application device according to claim 10, **characterised in that** the period of time is from 0.1 second to 5 seconds long.

12. High-frequency current application device according to claim 1, **characterised in that** the control device is designed to maintain the reduced output power for a minimum time independently of the variation over time of the impedance or resistance.

13. High-frequency current application device according to claim 12, **characterised in that** the minimum time is from 1 second to 10 seconds long.

14. High-frequency current application device according to claim 13, **characterised in that** the control device is designed to use the first derivative of the variation over time of the impedance or resistance again for the further control of the power output once the minimum time has expired.

15. High-frequency current application device according to claim 1 or 14, **characterised in that** the control device is designed to increase the output power again when the signal of the measuring and calculating device indicates that the first time derivative of the variation of the impedance or ohmic resistance exceeds a value in the range from minus 10 ohms/second to minus 0.1 ohm/second.

16. High-frequency current application device according to claim 9 or 15, **characterised in that** different threshold values can be chosen for different types of tissue.

17. High-frequency current application device according to claim 1, **characterised in that** the control device is designed to continuously restore the output power to the original power over a short period of time.

18. High-frequency current application device according to claim 17, **characterised in that** the period of time is from 0.1 second to 5 seconds long.

19. High-frequency current application device according to any one of the preceding claims, **characterised in that** the high-frequency generator, the measuring and calculating device and the control device are accommodated in a common housing.

20. High-frequency current application device according to claim 1, **characterised in that** the two electrodes between which the impedance or ohmic resistance measurement is carried out are arranged on a shaft which is to be introduced into the body tissue.

21. High-frequency current application device according to claim 1, **characterised in that** the two electrodes between which the impedance or ohmic resistance measurement is carried out are arranged on two different shafts, both of which are to be introduced into the body tissue.

22. High-frequency current application device according to claim 1, **characterised in that** at least one of the two electrodes between which the impedance or ohmic resistance measurement is carried out is formed of a plurality of separate elements which are arranged either on the same shaft or on different shafts and are at the same electric potential due to an electrically conducting connection.

23. High-frequency current application device according to claim 1, **characterised in that** one of the two electrodes between which the impedance or ohmic resistance measurement is carried out is a neutral electrode which is to be applied to the surface of the skin.

24. High-frequency current application device according to claim 23, **characterised in that** the neutral electrode is formed of a plurality of separate elements which are to be applied to different skin surfaces and which are at the same electric potential due to an electrically conducting connection.

25. High-frequency application device according to any one of claims 1 to 24, **characterised in that** the high-frequency application device has a control connection for an external pump which is connected to the electrodes introduced into the body tissue.

26. High-frequency application device according to claim 25, **characterised in that** the high-frequency application device is connected to a pump which is designed to convey fluid into body tissue that is to be treated.

27. High-frequency application device according to either claim 25 or claim 26, **characterised in that** the control device is designed to cause the pump to be switched on when the control device detects imminent tissue desiccation.

28. High-frequency application device according to claim 27, **characterised in that** the control device is designed to control the external pump so that fluid is conveyed into the treatment volume only by those electrodes between which the control device has detected imminent tissue desiccation.

29. High-frequency application device according to claim 27 or 28, **characterised in that** the control device is designed to cause the pump to be switched on and the output power to be reduced at the same time.

30. High-frequency application device according to claims 25 to 29, **characterised in that** the control device is designed to control the pump so that the pump conveys a previously defined amount of fluid into the treatment volume, the amount of fluid being such that it corresponds approximately to the amount of tissue water previously evaporated.

31. High-frequency application device according to claim 26, **characterised in that** the pump is designed to work with a constant delivery volume and the control device is designed to deactivate the external pump again when there is adequate tissue rehydration.

32. High-frequency application device according to claim 31, **characterised in that** the control device is designed to deactivate the pump and to restore the output power to the original power at the same point in time.

33. High-frequency application device according to claim 26, **characterised in that** the fluid is sterile physiological saline solution.

34. High-frequency application device according to claim 26, **characterised in that** the fluid is sterile water.

## Revendications

1. Dispositif d'application d'un courant de haute fréquence destiné à scléroser par voie thermique un tissu corporel, ledit dispositif comportant un générateur de hautes fréquences auquel sont raccordées au moins deux électrodes qui sont configurées pour venir au contact du tissu corporel, ainsi qu'un dispositif de mesure et de calcul qui est configuré pour déterminer l'impédance ou la résistance ohmique entre les deux électrodes, entre lesquelles circule un courant thérapeutique de haute fréquence, à partir d'au moins deux des paramètres intensité du courant, tension, rapport de phases et puissance active ou au moyen d'un circuit de mesure indépendant du circuit de courant thérapeutique, et un dispositif de contrôle relié au dispositif de mesure et de calcul et destiné à modifier la puissance de sortie, dans lequel
- le dispositif de mesure et de calcul étant configuré pour former, lorsque l'on applique un courant de haute fréquence, la dérivée première à partir de la variation dans le temps de l'impédance ou de la résistance ohmique et pour délivrer un signal qui est fonction de la valeur de la dérivée première, et
- le dispositif de contrôle étant configuré pour réduire la puissance de sortie de façon significative lorsque le signal du dispositif de mesure et de calcul indique que la dérivée par rapport au temps de l'impédance ou de la résistance ohmique devient supérieure à un seuil positif qui est caractéristique d'un risque de dessèchement du tissu et
**caractérisé en ce que** le dispositif de contrôle est configuré pour
- ramener la puissance de sortie à la puissance d'origine lorsque le signal du dispositif de mesure et de calcul indique que la dérivée première de la variation dans le temps de l'impédance ou de la résistance ohmique devient supérieure dans le sens des valeurs positives à un seuil négatif qui est caractéristique d'une réhydratation suffisante du tissu corporel.

2. Dispositif d'application d'un courant de haute fréquence selon la revendication 1, **caractérisé en ce que** le dispositif de mesure et de calcul est configuré pour déduire à partir d'un courant thérapeutique de haute fréquence circulant entre les électrodes les paramètres nécessaires à la détermination de l'impédance et de la résistance ohmique.

3. Dispositif d'application d'un courant de haute fréquence selon la revendication 2, **caractérisé en ce que** le dispositif de contrôle est configuré pour réduire la puissance de sortie à une valeur de l'ordre de 2% à 50% de la puissance de sortie d'origine lorsque le signal du dispositif de mesure et de calcul indique que la dérivée première de l'impédance ou de la résistance ohmique devient supérieure au seuil positif.

4. Dispositif d'application d'un courant de haute fréquence selon la revendication 1, **caractérisé en ce que** le dispositif de mesure et de calcul est configurée pour déduire les paramètres nécessaires à la détermination de l'impédance ou de la résistance ohmique seulement au moyen d'un circuit de mesure indépendant du circuit de courant thérapeutique.

5. Dispositif d'application d'un courant de haute fréquence selon la revendication 4, **caractérisé en ce que** le dispositif de contrôle est configuré pour réduire la puissance de sortie à une valeur de l'ordre de 0% à 50% de la puissance de sortie d'origine lorsque le signal du dispositif de mesure et de calcul indique que la dérivée première de l'impédance ou de la résistance ohmique devient supérieure au seuil positif.

6. Dispositif d'application d'un courant de haute fréquence selon la revendication 4, **caractérisé en ce que** le circuit de mesure séparé fonctionne à une fréquence différente de la fréquence de fonctionnement de la source de courant thérapeutique de haute fréquence.

7. Dispositif d'application d'un courant de haute fréquence selon la revendication 1, **caractérisé en ce que** le dispositif de mesure et de calcul est configuré pour former la dérivée première par rapport au temps de la variation de l'impédance ou de la résistance ohmique à partir de deux valeurs de mesure temporellement successives de l'impédance ou de la résistance ohmique.

8. Dispositif d'application d'un courant de haute fréquence selon la revendication 1, **caractérisé en ce que** le dispositif de mesure et de calcul est configuré pour calculer une valeur moyenne d'un nombre de valeurs de mesures temporellement successives de l'impédance ou de la résistance ohmique, et pour former la dérivée première par rapport au temps de la variation de l'impédance ou de la résistance ohmique à partir de deux valeurs moyennes temporellement successives de l'impédance ou de la résistance ohmique.

9. Dispositif d'application d'un courant de haute fréquence selon la revendication 1, **caractérisé en ce que** le dispositif de contrôle est configuré pour réduire la puissance de sortie lorsque le signal du dispositif de mesure et de calcul indique que la dérivée première par rapport au temps de la variation de l'impédance ou de la résistance ohmique devient supérieure à une valeur de l'ordre de 5 ohms/seconde à 50 ohms/seconde.

10. Dispositif d'application d'un courant de haute fréquence selon la revendication 1, **caractérisé en ce que** le dispositif de contrôle est configuré pour réduire la puissance de sortie de façon continue sur un intervalle de temps court.

11. Dispositif d'application d'un courant de haute fréquence selon la revendication 10, **caractérisé en ce que** l'intervalle de temps est compris entre 0,1 seconde et 5 secondes.

12. Dispositif d'application d'un courant de haute fréquence selon la revendication 1, **caractérisé en ce que** le dispositif de contrôle est configuré pour maintenir la puissance de sortie réduite pendant un temps minimum indépendamment de la variation dans le temps de l'impédance ou de la résistance.

13. Dispositif d'application d'un courant de haute fréquence selon la revendication 12, **caractérisé en ce que** le temps minimum est compris entre 1 seconde et 10 secondes.

14. Dispositif d'application d'un courant de haute fréquence selon la revendication 13, **caractérisé en ce que** le dispositif de contrôle est configuré pour tenir compte à nouveau de la dérivée première de la variation dans le temps de l'impédance ou de la résistance au bout du temps minimum pour commander encore la puissance utile.

15. Dispositif d'application d'un courant de haute fréquence selon la revendication 1 ou 14, **caractérisé en ce que** le dispositif de contrôle est configuré pour augmenter de nouveau la puissance de sortie lorsque le signal du dispositif de mesure et de calcul indique que la dérivée première par rapport temps de la variation de l'impédance ou de la résistance ohmique devient supérieure à une valeur de l'ordre de moins 10 ohms/seconde à moins 0,1 ohm/seconde.

16. Dispositif d'application d'un courant de haute fréquence selon la revendication 9 ou 15, **caractérisé en ce que** l'on peut choisir des valeurs de seuil différentes pour différents types de tissu.

17. Dispositif d'application d'un courant de haute fréquence selon la revendication 1, **caractérisé en ce que** le dispositif de contrôle est configuré pour ramener la puissance de sortie à la puissance d'origine de façon continue sur un court intervalle de temps.

18. Dispositif d'application d'un courant de haute fréquence selon la revendication 17, **caractérisé en ce que** l'intervalle de temps est compris entre 0,1 seconde et 5 secondes.

19. Dispositif d'application d'un courant de haute fréquence selon l'une des revendications précédentes, **caractérisé en ce que** le générateur de hautes fréquences, le dispositif de mesure et de calcul et le dispositif de contrôle sont montés dans un boîtier commun.

20. Dispositif d'application d'un courant de haute fréquence selon la revendication 1, **caractérisé en ce que** les deux électrodes, entre lesquelles est effectuée la mesure de l'impédance ou de la résistance ohmique, sont disposées sur une tige qui est destinée à être introduite dans le tissu corporel.

21. Dispositif d'application d'un courant de haute fréquence selon la revendication 1, **caractérisé en ce que** les deux électrodes, entre desquelles est effectuée la mesure de l'impédance ou de la résistance ohmique, sont disposées sur deux tiges différentes qui sont toutes les deux destinées à être introduites dans le tissu corporel.

22. Dispositif d'application d'un courant de haute fréquence selon la revendication 1, **caractérisé en ce que** au moins une des deux électrodes, entre lesquelles est effectuée la mesure de l'impédance ou de la résistance ohmique, est formée de plusieurs éléments séparés qui sont disposés soit sur la même tige soit sur des tiges différentes et qui sont portés au même potentiel électrique par une liaison électriquement conductrice.

23. Dispositif d'application d'un courant de haute fréquence selon la revendication 1, **caractérisé en ce que** l'une des deux électrodes, entre lesquelles est effectuée la mesure de l'impédance ou de la résistance ohmique, est une électrode neutre destinée à être appliquée sur la surface de la peau.

24. Dispositif d'application d'un courant de haute fréquence selon la revendication 23, **caractérisé en ce que** l'électrode neutre est formée de plusieurs éléments séparés qui sont destinés à être appliqués sur des surfaces de peau différentes et qui sont portés au même potentiel électrique par une liaison électriquement conductrice.

25. Dispositif d'application à haute fréquence selon l'une des revendications 1 à 24, **caractérisé en ce que** le dispositif d'application à haute fréquence comporte une borne de commande destinée à une pompe externe reliée aux électrodes introduites dans le tissu corporel.

26. Dispositif d'application à haute fréquence selon la revendication 25, **caractérisé en ce que** le dispositif d'application à haute fréquence est relié à une pompe qui est configurée pour transporter un liquide dans un tissu corporel à traiter.

27. Dispositif d'application à haute fréquence selon l'une des revendications 25 ou 26, **caractérisé en ce que** le dispositif de contrôle est configuré pour ordonner une mise sous tension de la pompe lorsque le dispositif de contrôle détecte un risque de dessèchement du tissu.

28. Dispositif d'application à haute fréquence selon la revendication 27, **caractérisé en ce que** le dispositif de contrôle est configuré pour commander la pompe externe de sorte qu'un liquide est transporté dans le volume de traitement exclusivement par des électrodes entre lesquelles le dispositif de contrôle a détecté un risque de dessèchement du tissu.

29. Dispositif d'application à haute fréquence selon la revendication 27 ou 28, **caractérisé en ce que** le dispositif de contrôle est configuré pour ordonner simultanément la mise sous tension de la pompe et la réduction de la puissance de sortie.

30. Dispositif d'application à haute fréquence selon l'une des revendications 25 à 29, **caractérisé en ce que** le dispositif de contrôle est configuré pour commander la pompe de sorte que la pompe transporte dans le volume de traitement une quantité de liquide prédéfinie, la quantité de liquide étant mesurée de façon à correspondre à peu près à la quantité d'eau tissulaire qui s'est évaporée.

31. Dispositif d'application à haute fréquence selon la revendication 26, **caractérisé en ce que** la pompe est configurée pour fonctionner avec un débit de transport constant et le dispositif de contrôle est configuré pour désactiver à nouveau la pompe externe lorsque la réhydratation du tissu est suffisante.

32. Dispositif d'application à haute fréquence selon la revendication 31, **caractérisé en ce que** le dispositif de contrôle est configuré pour désactiver la pompe et ramener la puissance de sortie à la puissance d'origine au même instant.

33. Dispositif d'application à haute fréquence selon la revendication 26, **caractérisé en ce que** le liquide est une solution saline physiologique stérile.

34. Dispositif d'application à haute fréquence selon la revendication 26, **caractérisé en ce que** le liquide est de l'eau stérile.
